# EUROPÄISCHE PATENTANMELDUNG

(11) **EP 1 346 744 A1**
(43) Veröffentlichungstag der Anmeldung: **24.09.2003**
(21) Anmeldenummer: 02006473.9
(22) Anmeldetag: 22.03.2002
(51) Int. Cl.: A61M 25/00

(54) **Katheteranordnung zur Stentimplantation**

(71) Anmelder: JOMED NV, 6235 NS Ulestraten (NL)
(72) Erfinder: Calisse, Jorge, Dr., 10785 Berlin (DE)
(74) Vertreter: Schmitz, Hans-Werner, Dipl.-Ing.

(57) **Zusammenfassung**

Um das Ziel einer hohen Flexibilität und gleichzeitig Tragfähigkeit eines Führungsdrahttubus (3) einer erfindungsgemäßen Katheteranordnung zu erreichen, weist das distale Ende (5) des Führungsdrahttubus (3) Wandausnehmungen (11) auf, wobei das distale Ende (5) des Führungsdrahttubus (3) aus dem gleichen festen Material wie das proximale Ende (4) besteht.

## Beschreibung

Die vorliegende Erfindung betrifft eine Katheteranordnung nach dem Oberbegriff des Anspruchs 1.

An Verengungsstellen in Körpergefäßen oder Körperhöhlungen werden heutzutage zur Aufweitung der Verengung Katheteranordnungen verwendet, welche an ihrem distalen Ende einen Ballon aufweisen, welcher expandierbar ist. Auf diesen expandierbaren Ballon kann zusätzlich ein Stent aufgecrimpt sein, der zur Stabilisierung der Gefäßwand in der Verengung platziert werden kann. Die Katheteranordnung wird dann mit Hilfe eines Führungsdrahtes an die verengte Stelle im Körper des Patienten geführt und die Verengung durch Expansion des Ballons aufgeweitet beziehungsweise der aufgecrimpte Stent in der Verengung platziert. Ferner werden derartige Katheteranordnungen auch mit Ultraschallköpfen ausgerüstet, was ein festes Material, insbesondere für den Führungsdrahttubus, bedingt, da es ansonsten zum Ausknicken des Katheters kommen kann.

Es ist daher Aufgabe der vorliegenden Erfindung eine Katheteranordnung der im Oberbegriff des Anspruchs 1 angegebenen Art zu schaffen, welche eine besonders hohe Flexibilität am distalen Endbereich und eine ausreichende Festigkeit für die Anbringung eines Ultraschallkopfes aufweist.

Die Lösung dieser Aufgabe erfolgt durch die Merkmale des Anspruchs 1. Vorteilhafte Weiterbildungen sind Gegenstand der Unteransprüche.

Aus dem US-Patent 5,599,319 ist zwar ein Stent mit einer weichen Spitze bekannt, jedoch wird bei diesem Stent die weiche Spitze durch einen Materialübergang auf ein Material geringerer Festigkeit erreicht. In diesem Bereich sind darüber hinaus Ringe angeordnet, was das Problem aufwirft, dass dieser Bereich, insbesondere beim Anbringen von Ultraschallköpfen, zum Ausknicken neigt.

Die Qualität eines Ballonkatheters zeichnet sich dadurch aus, dass der Katheter möglichst einfach gewundenen Gefäßabschnitten folgen kann. Wird daher bevorzugend die distale Spitze - im Allgemeinen "Tip" genannt - aus einem hierfür besonders geeigneten biegeweicheren Material gestaltet, so folgt das gesamte System bevorzugt den Windungen des Gefäßes. Gleichzeitig muss jedoch gewährleistet sein, dass der Katheter in die verengten Bereiche des Gefäßes sicher vorgeschoben werden kann.

Diese Eigenschaft wird durch den Fachbegriff "pushability" beschrieben. Sofern jedoch das gesamte Kathetersystem aus weichen Materialien gefertigt wird, wirkt dies kontraindikativ.

Erfindungsgemäß besteht der Führungsdrahttubus der Katheteranordnung über seine gesamte Länge aus dem selben Material. Dabei ist jedoch der Teil des Führungsdrahttubus, welcher das distale Ende bildet, mit Wandausnehmungen versehen, was zu einer größeren Elastizität bzw. Flexibilität dieses Bereiches führt. Dadurch kann eine besonders flexible Ausgestaltung des distalen Endes des Führungsdrahttubus bzw. der Katheteranordnung erreicht werden. Die Katheteranordnung erhält dadurch eine größere Biegefähigkeit unter gleichzeitiger Erhaltung der "pushability".

Vorteilhafter Weise können die Wandausnehmungen Wanddurchbrechungen sein, die die gesamte Wandstärke des distalen Endes durchgreifen.

Es ist hierbei ferner vorteilhafter Weise möglich, unterschiedliche Anordnungsmuster für die Wandausnehmungen vorzusehen.

Weitere Einzelheiten, Merkmale und Vorteile der Erfindung ergeben sich aus der nachfolgenden Beschreibung von Ausführungsbeispielen anhand der Zeichnung.

Es zeigt:
- Fig. 1: eine schematisch stark vereinfachte Schnittdarstellung einer erfindungsgemäßen Katheteranordnung gemäß einem ersten Ausführungsbeispiel der vorliegenden Erfindung;
- Fig. 2: eine schematisch stark vereinfachte Schnittdarstellung einer erfindungsgemäßen Katheteranordnung gemäß Fig. 1 im Bereich des distalen Endes in vergrößertem Maßstab, und
- Fig. 3: eine schematisch stark vereinfachte Darstellung eines weiteren Ausnehmungsmusters im Bereich des distalen Endes.

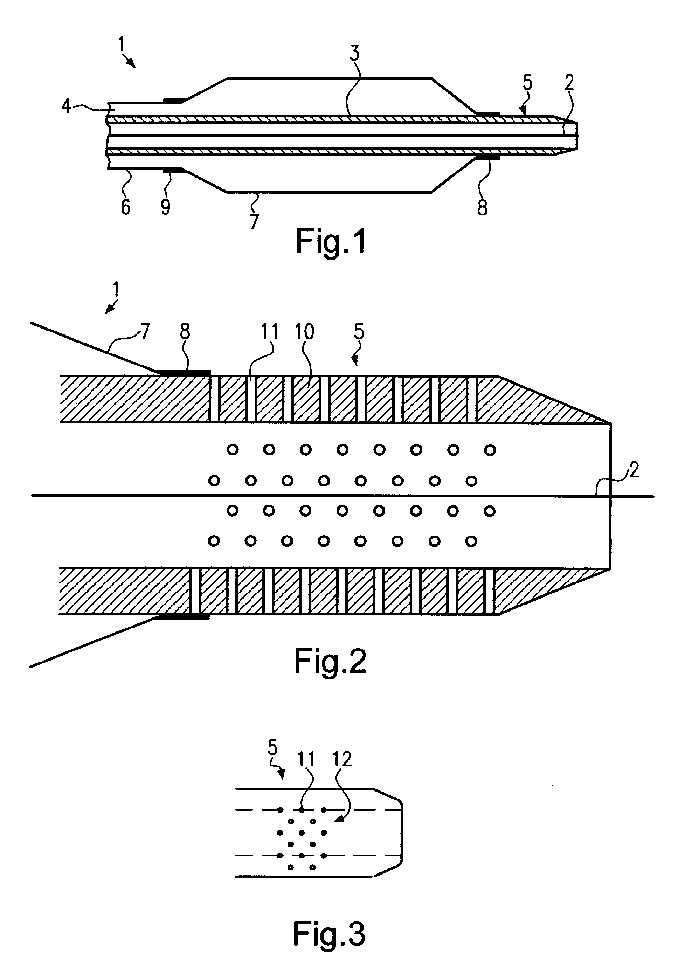

In Fig. 1 ist eine Katheteranordnung 1 zur Implantation eines Stents dargestellt. Die Katheteranordnung 1 weist einen Führungsdrahttubus 3 auf, in dem ein Führungsdraht 2 angeordnet ist. Der Führungsdraht 2 und der Führungsdrahttubus 3 bilden gemeinsam eine Führungseinrichtung.

Wie in Fig. 1 gezeigt, umfasst die Katheteranordnung 1 weiter einen Ballon 7 und einen Tubus 6. Der Ballon 7 ist mit seinem distalen Ende 5 über einen distalen Befestigungsbereich 8 am Führungsdrahttubus 3 befestigt und mit seinem proximalen Ende 4 über einen proximalen Befestigungsbereich 9 am Tubus 6 befestigt (vgl. Fig. 1). Hierbei wird unter "distalem Ende" "zum Herzen hinführend" und unter "proximalem Ende" "vom Herzen wegführend" verstanden. Der mit dem Ballon 7 verbundene Tubus 6 dient dazu, um ein geeignetes Medium wie z.B. eine Kochsalzlösung in den hohlen Innenraum des Ballons 7 zuzuführen und diesen an der verengten Stelle zu expandieren. Dadurch kann ein um den Ballon 7 angebrachter Stent (nicht dargestellt) expandiert werden und seinerseits die Aufweitung der verengten Stelle im Körperhohlgefäß als Gefäßstütze stabilisieren.

Wie aus Fig. 1 ersichtlich ist, ist der distale Befestigungsbereich 8 des Ballons 7 am Führungsdrahttubus 3 etwas in proximaler Richtung vom distalen Ende des Führungsdrahttubus versetzt angeordnet. Dadurch ist die Verbindungsstelle zwischen Ballon 7 und Führungsdrahttubus 3 versetzt vom Ende des Führungsdrahttubus 3 angeordnet.

Die erfindungsgemäße Katheteranordnung weist im Gegensatz zu bekannten Katheteranordnungen einen weichen Spitzenbereich auf, dessen Flexibilität beziehungsweise Elastizität größer ist als der verbleibende Teil des Führungsdrahttubus 3. Dadurch können Verletzungen des Patienten beim Einführen der Katheteranordnung verhindert werden.

In Fig. 2 ist der distale Endbereich 5 in vergrößerter Darstellung wiedergegeben. Aus dieser Darstellung wird deutlich, dass die Wand 10 dieses distalen Endbereiches 5 mit einer Mehrzahl von Wandausnehmungen versehen ist, von denen repräsentativ eine mit der Bezugsziffer 11 gekennzeichnet ist.

Bei der besonders bevorzugten Ausführungsform gemäß Fig. 2 durchgreifen die Wandausnehmungen 11 die gesamte Wandstärke der Wand 10. Prinzipiell ist es jedoch auch möglich, dass Wandausnehmungen 11 vorgesehen sind, deren Tiefe geringer ist als die Wandstärke.

Durch diese Wandausnehmungen 11 ergibt sich eine erhöhte Flexibilität des distalen Endbereiches 5, obwohl dieser aus dem gleichen festen Material wie der proximale Bereich 4 des Führungsdrahttubus 3 besteht.

Fig. 3 verdeutlicht in schematisch stark vereinfachter Ausführungsform, dass unterschiedliche Anordnungsmuster 12 für die Wandausnehmungen 11 denkbar sind, wobei diese Darstellung jedoch lediglich beispielhaft ist. Prinzipiell kann die Art des Ausnehmungsmusters 12 an den jeweiligen Anwendungsfall angepasst werden.

Nachzutragen ist ferner, dass die Wandausnehmungen 11 als Einzelkanäle ausgebildet sind, deren jeweilige Längsachse zumindestens im Wesentlichen senkrecht zur Längsmittellinie des distalen Endes 5 verläuft. Insbesondere handelt es sich bei diesen Ausnehmungen 11 nicht um um die gesamte Spitze 5 umlaufende Ringe.

## Patentansprüche

1. Katheteranordnung mit
- einem Führungsdrahttubus (3) zur Aufnahme eines Führungsdrahts (2) und
- einem am Führungsdrahttubus (3) befestigten, expandierbaren Ballon (7), wobei das distale Ende (5) des Führungsdrahttubus (3) über das distale Ende des Ballons (7) hinaussteht,
**dadurch gekennzeichnet,**
**dass** das distale Ende (5) des Führungsdrahttubus (3) aus dem selben festen Material wie der proximale Teil (4) des Führungsdrahttubus (3) besteht und dass das distale Ende (5) mit Wandausnehmungen (11) versehen ist.

2. Katheteranordnung nach Anspruch 1, **dadurch gekennzeichnet, dass** die Wandausnehmungen (11) die gesamte Wandstärke der Wand (10) des distalen Endes (5) durchgreifen.

3. Katheteranordnung nach Anspruch 1, **dadurch gekennzeichnet, dass** die Wandausnehmungen (11) nur einen Teil der Wand (10) des distalen Endes (5) durchgreifen.

4. Katheteranordnung nach einem der Ansprüche 1 bis 3, **dadurch gekennzeichnet, dass** die Wandausnehmungen (11) in unterschiedlichen Ausnehmungsmustern (12) angeordnet sind.
